# EUROPEAN PATENT APPLICATION

(11) **EP 4 617 261 A1**
(43) Date of publication of application: **17.09.2025**
(21) Application number: 23887858.1
(22) Date of filing: 31.10.2023
(51) Int. Cl.: C07D 239/48, C07D 401/12, C07D 405/12, C07D 403/12, A61P 35/00, A61P 35/02, A61K 31/506, A61K 31/551

(54) **2,4,5-TRISUBSTITUTED PYRIMIDINE COMPOUND, PREPARATION METHOD THEREFOR, AND USE THEREOF**

(30) Priority: 11.11.2022 CN 202211413247
(71) Applicant: Wenzhou Medical University, Wenzhou, Zhejiang 325035 (CN)
(72) Inventor: LI, Xiaokun, Wenzhou, Zhejiang 325035 (CN); LIU, Zhiguo, Wenzhou, Zhejiang 325035 (CN); LIN, Li, Wenzhou, Zhejiang 325035 (CN); ZHENG, Xiaohui, Wenzhou, Zhejiang 325035 (CN)
(74) Representative: karo IP
(86) International application number: PCT/CN2023/128270
(87) International publication number: WO 2024/099168

(57) **Abstract**

Disclosed is a 2,4,5-trisubstituted pyrimidine compound having a structure as represented by the formula (I) compound, an isotope labeled compound, or an optical isomer, a geometric isomer, a tautomer or an isomer mixture thereof, or a pharmaceutically acceptable salt thereof, or a prodrug thereof, or a metabolite thereof: 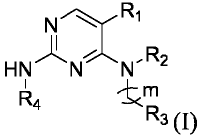 wherein R₁ can be selected from one of halogen, alkyl, or alkoxy; R₂ can be selected from any one of H or C₁-C₂ alkyl; and a plurality of substituents can be selected for R₃ and R₄. The compound can inhibit FGFR1 and can be used as a potential anti-tumor drug.

## Description

### TECHNICAL FIELD

The present disclosure belongs to the field of pharmaceutical chemistry, and particularly relates to a 2,4,5-trisubstituted pyrimidine compound targeting FGFR1, a preparation method therefor, and a use thereof.

### BACKGROUND

In the field of protein kinases, a fibroblast growth factor receptor 1 (FGFR1) family is a very attractive target in tumor treatment. The FGFR1 family of receptor tyrosine kinases consists of four TK receptors: FGFR1-FGFR4, which exhibit a high degree of sequence homology. Overexpression of FGFR1 kinase is closely associated with various malignant tumors, such as breast cancer, bladder cancer, gastric cancer, prostate cancer, and colorectal cancer. Therefore, researchers of oncological medicine spare continuous efforts to investigate FGFR1 to use it for cancer diagnosis and treatment. A variety of methods can be used to inhibit disease-related protein kinases, block and destroy signal transduction of tumor cells. However, since synthetic antisense oligonucleotides are susceptible to degradation by ribozymes, and RNA interference technology faces challenges related to safety, stability, and off-target effects, drug developers have attempted to screen small molecule inhibitors of FGFR1 from organically-synthesized small molecules. To date, four FGFR inhibitors available on the market are erdafitinib, pemigatinib, infigrarinib and TAS-120. Specifically, TAS-120 is a first approved irreversible FGFR inhibitor, and is used for treating patients with locally advanced or metastatic cholangiocarcinoma carrying FGFR2 gene rearrangements (including gene fusion). The successful application of covalent bonds has provided people with design inspiration, as irreversible binding is considered a practical mechanism for situations where reversible inhibitors are ineffective for drug-resistant mutants and tyrosine kinases having high affinity with adenosine triphosphate (ATP). Therefore, irreversible inhibitors targeting FGFR1 have great prospects. Currently, developing a novel FGFR1 inhibitor capable of overcoming the aforementioned limitations is an urgent problem to be solved.

### SUMMARY

One objective of the present disclosure is to address the above technical problems by providing a 2,4,5-trisubstituted pyrimidine compound targeting FGFR1, a preparation method therefor, and a use thereof. The compound can selectively inhibit the phosphorylation of FGFR kinase, and thus can be used for treating malignant tumors closely associated with the kinase.

The 2,4,5-trisubstituted pyrimidine compound has the following general structural formula:: 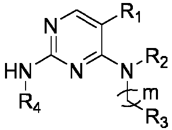
where R₁ is selected from one of halogen, trifluoromethyl, C₁-C₆ alkyl, or C₁-C₆ alkoxy;
R₂ is selected from any one of H or C₁- C₅ alkyl;
where m is 0 or 1 or 2;
Preferably, R₁ can be selected from any one of F, Cl, CH₃, OCH₃ or CF₃;
Preferably, R₂ can be selected from any one of H, CH₃, or CH₂CH₃;
Preferably, m=0;
R₃ is selected from any of the following substituents: 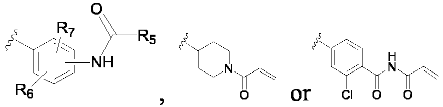
where R₆ is selected from Cl or methyl; R₇ is selected from H or Cl; R₅ is selected from vinyl, propenyl, butenyl, trifluorovinyl, methyl, ethyl, phenyl, tetrahydrofuran-2-yl, or acrylamide.

R₃ is further selected from any of the following substituents:

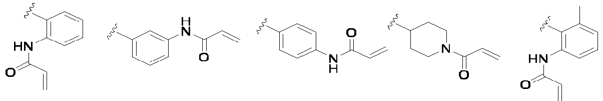

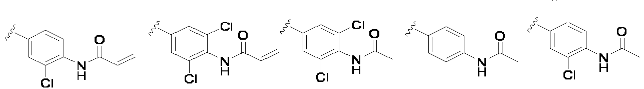

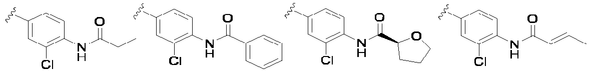

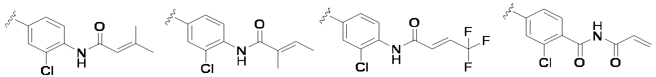

R₄ is selected from any of the following substituents:

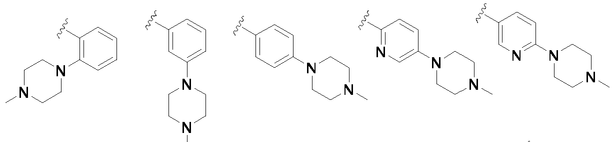

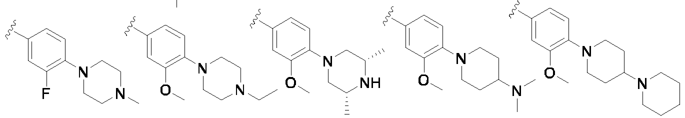

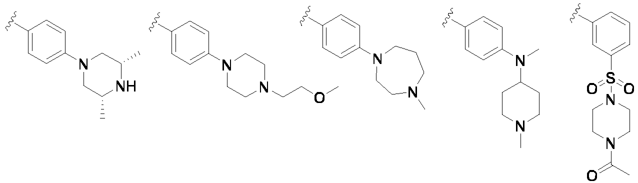

The substituents in the above R₁, R₂, R₃, and R₄ can be combined in any manner.

Preferably, the R₁ is Cl;
the R₂ is H;
m is 0;
the R₃ is 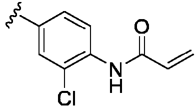 ; and
the R₄ is selected from any of the following substituents:

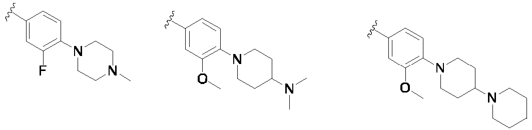

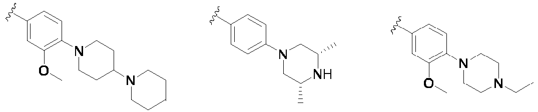

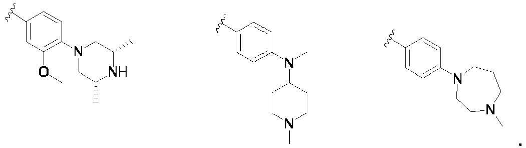

The compound in the present disclosure may exist in the form of a pharmaceutically acceptable salt thereof.

The pharmaceutically acceptable salt thereof include acid addition salts and base addition salts thereof. Suitable acid addition salts are formed from acids that form non-toxic acids. Examples of the acid addition salts include but are not limited to: acetate, adipate, aspartate, benzoate, benzenesulfonate, bicarbonate/carbonate, bisulfate/sulfate, borate, camphorsulfonate, citrate, cyclohexylamine sulfonate, ethanedisulfonate, formate, fumarate, glucoheptonate, gluconate, glucuronate, hexafluorophosphate, 2-(4-hydroxybenzyl)benzoate, hydrochloride/chloride, hydrobromide/bromide, hydroiodide/iodide, 2-hydroxyethanesulfonate, lactate, malate, maleate, malonate, methanesulfonate, methylsulfate, naphthoate, 2-naphthalenesulfonate, nicotinate, nitrate, orotate, oxalate, hexadecanoate, phosphate/hydrogen phosphate/dihydrogen phosphate, pyroglutamate, glucarate, stearate, salicylate, tannate, tartrate, toluenesulfonate, and trifluoroacetate. Suitable base addition salts are formed from bases that form non-toxic acids. Examples of the base addition salts include but are not limited to: aluminum, arginine, calcium, choline, diethylamine, diethanolamine, glycine, lysine, magnesium, meglumine, ethanolamine, potassium, sodium, tromethamine, and zinc salts. Half-salts of acids and bases, such as hemisuccinate and hemicalcium salts, can also be formed.

The present disclosure further provides a use of the 2,4,5-trisubstituted pyrimidine compound in the preparation of a drug, where the drug is an anti-tumor drug.

Preferably, the anti-tumor drug targets FGFR1.

Preferably, the drug is used to treat one or more of hepatocellular carcinoma, breast cancer, bladder cancer, colorectal cancer, melanoma, mesothelioma, lung cancer, prostate cancer, pancreatic cancer, testicular cancer, thyroid cancer, squamous cell carcinoma, glioblastoma, fibrosarcoma, uterine cancer, rhabdomyosarcoma, skin cancer, kidney cancer, lymphoma, cervical cancer, head and neck squamous cell carcinoma, malignant hydatidiform mole, choriocarcinoma, ovarian cancer, gastric cancer, and leukemia. Further preferably, the drug is used to treat non-small cell lung cancer.

### BRIEF DESCRIPTION OF THE DRAWINGS

FIG. 1 shows inhibitory activity of some compounds in Example 9 against a wild-type FGFR1.
FIG. 2 shows inhibitory activity of some compounds in Example 9 against an FGFR1 mutant V561M.
FIG. 3 shows inhibitory activity of some compounds in Example 9 against an FGFR1 mutant V561F.

### DETAILED DESCRIPTIONS OF THE EMBODIMENTS

The examples described below are for illustrative purposes only, and are used to illustrate various aspects and embodiments of the present disclosure, but are not intended to limit the scope of protection of the present disclosure in any way.

All starting materials and reagents are commercially available and used directly without further purification, unless otherwise specified. The instruments and equipment used in the synthesis experiments and product analysis are all conventional instruments and equipment commonly used in organic synthesis.

¹H-NMR spectra are measured using a Bruker instrument (400MHz), and chemical shifts are expressed in ppm. ¹H NMR notation is expressed as follows by using tetramethylsilane as an internal standard (0.00ppm): s = singlet, d = doublet, t = triplet, m = multiplet, br = broad, dd = double doublet, dt = double triplet. When a coupling constant is provided, a unit is Hz.

The mass spectra are measured using a high-resolution mass spectrometer, and an ionization method can be electrospray ionization (ESI).

The purity is determined using high-performance liquid chromatography (Agilent 1260), and the mobile phase consists of chromatographic methanol and an ultrapure water system.

Hydrogen and nitrogen environments refer to a reaction vessel connected to a balloon with a volume of about 1 L.

Unless otherwise specified in the examples, the solution in the reactions refers to an aqueous solution.

### Example 1: Preparation of

### N-(4-((5-fluoro-2-((4-(4-methylpiperazine-1-yl)phenyl)amino)pyrimidine-4-yl)amino)phenyl)acr ylamide (Compound 1)

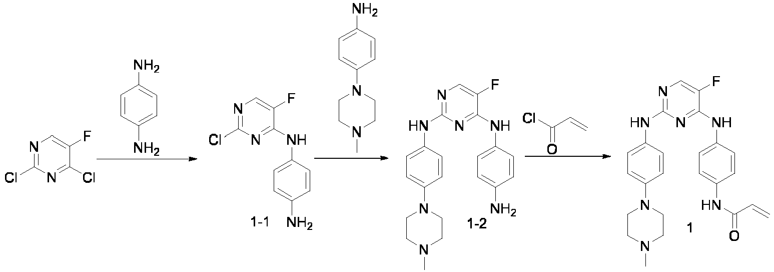

### Synthesis of an intermediate 1-1:

### N¹-(2-chloro-5-fluoropyrimidin-4-yl)benzene-1,4-diamine

2,4-dichloro-5-fluoropyrimidine (166.0 mg, 1.00 mmol), p-phenylenediamine (129.7 mg, 1.20 mmol), and anhydrous sodium carbonate (318.0 mg, 3.00 mmol) were fully dissolved in anhydrous ethanol (10 mL) to obtain a mixture, the mixture was condensed and refluxed at 80°C, and was distilled under reduced pressure for reaction, the reaction was completed after 3 h, the mixture was distilled under reduced pressure, extracted with ethyl acetate and concentrated under reduced pressure, and then separated and purified by column chromatography to obtain a white solid Compound 1-1, with a yield of 88.8%.

### Synthesis of an intermediate 1-2:

### N⁴-(4-aminophenyl)-5-fluoro-N²-(4-(4-methylpiperazine-1-yl)phenyl)pyrimidine-2,4-diamine

Compound 1-1 (210.7 mg, 0.88 mmol) and 4-(4-ethylpiperazine-1-yl)aniline (201.9 mg, 1.06 mmol) were fully dissolved in 2-butanol (10 mL), and trifluoroacetic acid (196 µL, 2.64 mmol) was added dropwise to obtain a mixture, the mixture was condensed and refluxed at 100 °C for reaction, the reaction was completed after 8 h, the mixture was distilled under reduced pressure, extracted with ethyl acetate and concentrated under reduced pressure, and then separated and purified by column chromatography to obtain a light brown solid Compound 1-2, with a yield of 40.6%.

### Synthesis of Compound 1:

### N-(4-((5-fluoro-2-((4-(4-methylpiperazine-1-yl)phenyl)amino)pyrimidine-4-yl)amino)phenyl)acr ylamide

Compound 1-2 (137.7 mg, 0.35 mmol) was fully dissolved in anhydrous tetrahydrofuran (5 mL), and anhydrous sodium carbonate (111.3 mg, 1.05 mmol) was added and stirred for 5 min at 0°C to obtain a mixture, acryloyl chloride (44 µL, 0.53 mmol) was added dropwise to the mixture for reaction, the reaction was completed after 1 h, the mixture was then quenched with NH₄Cl solution, distilled under reduced pressure, extracted with ethyl acetate and concentrated under reduced pressure, and then separated and purified by column chromatography to obtain a gray-green solid Compound 1, with a yield of 64.1%.

### Example 2:

### N-(4-(2-(5-chloro-2-((4-(4-methylpiperazine-1-yl)phenyl)amino)pyrimidine-4-yl)amino)ethyl)ph enyl)acrylamide (Compound 33)

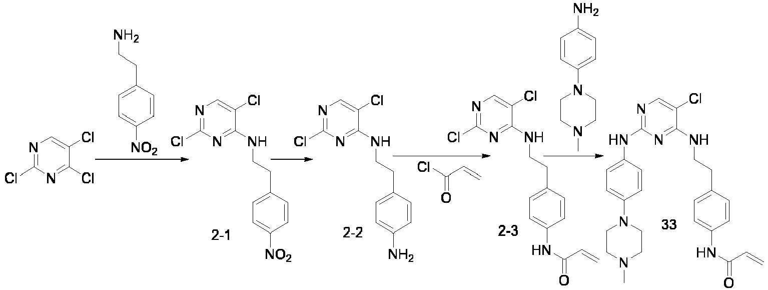

### Synthesis of an intermediate 2-1: 2,5-dichloro-N-(4-nitrophenethyl)pyrimidine-4-amine

2,4,5-trichloropyrimidine (167.0 mg, 1.00 mmol), 2-(4-nitrophenyl)ethane-1-amine hydrochloride (242.5 mg, 1.20 mmol), and anhydrous sodium carbonate (318.0 mg, 3.00 mmol) was fully dissolved in anhydrous ethanol (10 mL), and stirred at room temperature for reaction, the reaction was completed after 2 h to obtain a mixture, the mixture was then distilled under reduced pressure, extracted with ethyl acetate and concentrated under reduced pressure, and then separated and purified by column chromatography to obtain a light yellow solid Compound 2-1, with a yield of 87.0%.

### Synthesis of an intermediate 2-2:

### N-(4-aminophenethyl)-2,5-dichloropyrimidine-4-amine

Compound 2-1 (271.5 mg, 0.87 mmol) and anhydrous ammonium chloride (234.9 mg, 4.35 mmol) were fully dissolved in a mixed solution (EtOH:H₂O=3 : 1, 10 mL) to obtain a mixture, the mixture was condensed and refluxed at 60°C for 30 min, followed by the addition of iron powder (48.7 mg, 4.35 mmol) and heating to 80°C for reaction, the reaction was completed after 3 h, the mixture was then distilled under reduced pressure, filtered and extracted with ethyl acetate, and concentrated under reduced pressure, and then separated and purified by column chromatography to obtain a yellow solid Compound 2-2, with a yield of 95.1%.

### Synthesis of an intermediate 2-3:

### N-(4-(2-(2,5-dichloropyrimidine-4-yl)amino)ethyl)phenyl)acrylamide

Compound 2-2 (225.0 mg, 0.80 mmol) was fully dissolved in anhydrous tetrahydrofuran (5 mL), and anhydrous sodium carbonate (254.4 mg, 2.40 mmol) was added and stirred for 5 min at 0°C to obtain a mixture, acryloyl chloride (100 µL, 1.20 mmol) was added dropwise to the mixture for reaction, the reaction was completed after 1 h, the mixture was then quenched with NH₄Cl solution, distilled under reduced pressure, extracted with ethyl acetate and concentrated under reduced pressure, and then separated and purified by column chromatography to obtain a solid Compound 2-3, with a yield of 72.2%.

### Synthesis of Compound 33:

### N-(4-(2-(5-chloro-2-((4-(4-methylpiperazine-1-yl)phenyl)amino)pyrimidine-4-yl)amino)ethyl)ph enyl)acrylamide

Compound 2-3 (168.0 mg, 0.50 mmol) and 4-(4-ethylpiperazine-1-yl)aniline (114.8 mg, 0.60 mmol) were fully dissolved in 2-butanol (5 mL), and trifluoroacetic acid (75 µL, 1.00 mmol) was added dropwise to obtain a mixture, the mixture was condensed and refluxed at 100 °C for reaction, the reaction was completed after 8 h, the mixture was distilled under reduced pressure, extracted with ethyl acetate and concentrated under reduced pressure, and then separated and purified by column chromatography to obtain a light yellow solid Compound 33, with a yield of 37.3%.

### Example 3:

### N-((5-chloro-2-(4-(4-methylpiperazine-1-yl)phenyl)amino)pyrimidine-4-yl)amino)-3-methylphe nyl)acrylamide (Compound 11)

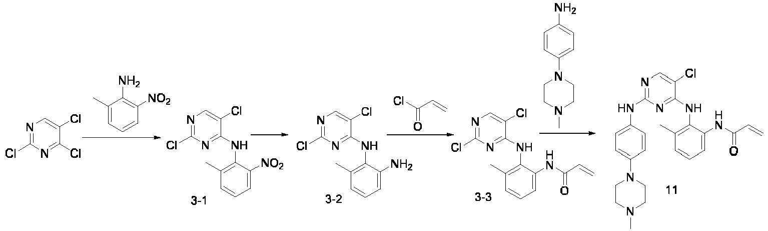

### Synthesis of an intermediate 3-1:

### 2,5-dichloro-N-(2-methyl-6-nitrophenyl)pyrimidine-4-amine

2-methyl-6-nitroaniline (182.5 mg, 1.20 mmol) was dissolved in anhydrous dimethylformamide (DMF) (5 mL) and stirred thoroughly at 0°C, sodium hydride (NaH, 72.0 mg, 3.00 mmol) was dissolved in anhydrous DMF (1 mL) and mixed well, which was then added dropwise to a reaction system for activating for 30 min, 2,4,5-trichloropyrimidine (167.0 mg, 1.00 mmol) was then added for reaction, the reaction was completed after 1 h to obtain a mixture, the mixture was then extracted with ethyl acetate, and concentrated under reduced pressure, and then separated and purified by column chromatography to obtain a white solid Compound 3-1, with a yield of 76.2%.

### Synthesis of an intermediate 3-2:

### N¹(2,5-dichloropyrimidin-4-yl)-6-methylbenzen-1,2-diamine

Compound 3-1 (208.6 mg, 0.70 mmol) and anhydrous ammonium chloride (189.0 mg, 3.50 mmol) were fully dissolved in a mixed solution (EtOH : H₂O=3 : 1, 8 mL) to obtain a mixture, the mixture was condensed and refluxed at 60°C for 30 min, followed by the addition of iron powder (196.0 g, 3.50 mmol) and heating to 80°C for reaction, the reaction was completed after 3 h, the mixture was then distilled under reduced pressure, filtered and extracted with ethyl acetate, and concentrated under reduced pressure, and then separated and purified by column chromatography to obtain a light yellow solid Compound 3-2, with a yield of 90.9%.

### Synthesis of an intermediate 3-3:

### N-(2-((2,5-dichloropyrimidin-4-yl)amino)-3-methylphenyl)acrylamide

Compound 3-2 (134.0 mg, 0.50 mmol) was fully dissolved in anhydrous tetrahydrofuran (5 mL), and anhydrous sodium carbonate (159.0 mg, 1.50 mmol) was added and stirred for 5 min at 0°C to obtain a mixture, acryloyl chloride (60 µL, 0.75 mmol) was added dropwise to the mixture for reaction, the reaction was completed after 1 h, the mixture was then quenched with NH₄Cl solution, distilled under reduced pressure, extracted with ethyl acetate and concentrated under reduced pressure, and then separated and purified by column chromatography to obtain a solid Compound 3-3, with a yield of 94.6%.

### Synthesis of Compound 11:

### N-((5-chloro-2-(4-(4-methylpiperazine-1-yl)phenyl)amino)pyrimidine-4-yl)amino)-3-methylphe nyl)acrylamide

Compound 3-3 (144.9 mg, 0.45 mmol) and 4-(4-ethylpiperazine-1-yl)aniline (114.8 mg, 0.60 mmol) were fully dissolved in 2-butanol (5 mL), and trifluoroacetic acid (75 µL, 1.00 mmol) was added dropwise to obtain a mixture, the mixture was condensed and refluxed at 100 °C for reaction, the reaction was completed after 8 h, the mixture was distilled under reduced pressure, extracted with ethyl acetate and concentrated under reduced pressure, and then separated and purified by column chromatography to obtain a white solid Compound 11, with a yield of 16.7%.

### Example 4:

### 1-(4-((5-fluoro-2-((4-(4-methylpiperazine-1-yl)phenyl)amino)pyrimidine-4-yl)amino)piperidine-1-yl)prop-2-en-1-one (Compound 19)

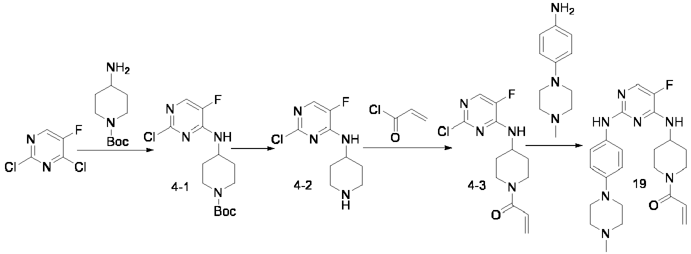

### Synthesis of an intermediate 4-1:

### 4-((2-chloro-5-fluoropyrimidine-4-yl)amino)piperidine-1-carboxylic acid tert-butyl ester

2,4-dichloro-5-fluoropyrimidine (166.0 mg, 1.00 mmol), 4-aminopiperidine-1-carboxylic acid tert-butyl ester (240.2 mg, 1.20 mmol), and anhydrous sodium carbonate (318.0 mg, 3.00 mmol) were fully dissolved in anhydrous ethanol (10 mL) to obtain a mixture, the mixture was condensed and refluxed at 70°C, the mixture was then distilled under reduced pressure for reaction, the reaction was completed after 2 h, the mixture was distilled under reduced pressure, extracted with ethyl acetate and concentrated under reduced pressure, and then separated and purified by column chromatography to obtain a solid Compound 4-1, with a yield of 82.1%.

### Synthesis of an intermediate 4-2:

### 2-chloro-5-fluoro-N-(piperidine-4-yl)pyrimidine-4-amine

Compound 4-1 (264.1 mg, 0.80 mmol) was dissolved in dichloromethane (4 mL), and stirred thoroughly at room temperature, 4 mol/L hydrochloric acid (HCI) solution was added dropwise for reaction, the reaction was completed after 30 min to obtain a mixture, the mixture was distilled under reduced pressure, extracted with ethyl acetate and concentrated under reduced pressure, and then separated and purified by column chromatography to obtain a solid Compound 4-2, with a yield of 85.3%.

### Synthesis of an intermediate 4-3:

### 1-(4-((2-chloro-5-fluoropyrimidine-4-yl)amino)piperidine-1-yl)prop-2-en-1-one

Compound 4-2 (147.0 mg, 0.60 mmol) was fully dissolved in anhydrous tetrahydrofuran (5 mL), and anhydrous sodium carbonate (159.0 mg, 1.50 mmol) was added and stirred for 5 min at 0°C to obtain a mixture, acryloyl chloride (75 µL, 0.90 mmol) was added dropwise to the mixture for reaction, the reaction was completed after 1 h, the mixture was then quenched with NH₄Cl solution, distilled under reduced pressure, extracted with ethyl acetate and concentrated under reduced pressure, and then separated and purified by column chromatography to obtain a light yellow solid Compound 4-3, with a yield of 90.4%.

### Synthesis of Compound 19:

### 1-(4-((5-fluoro-2-((4-(4-methylpiperazine-1-yl)phenyl)amino)pyrimidine-4-yl)amino)piperidine-1-yl)prop-2-en-1-one

Compound 4-3 (142.0 mg, 0.50 mmol) and 4-(4-ethylpiperazine-1-yl)aniline (114.8 mg, 0.60 mmol) were fully dissolved in 2-butanol (5 mL), and trifluoroacetic acid (75 µL, 1.00 mmol) was added dropwise to obtain a mixture, the mixture was condensed and refluxed at 100 °C for reaction, the reaction was completed after 8 h, the mixture was distilled under reduced pressure, extracted with ethyl acetate and concentrated under reduced pressure, and then separated and purified by column chromatography to obtain a white solid Compound 19, with a yield of 27.5%.

### Example 5: Preparation of

### N-(2-chloro-4-((5-fluoro-2-((4-(4-methylpiperazine-1-yl)phenyl)amino)pyrimidine-4-yl)(methyl) amino)phenyl)acetamide (Compound 27

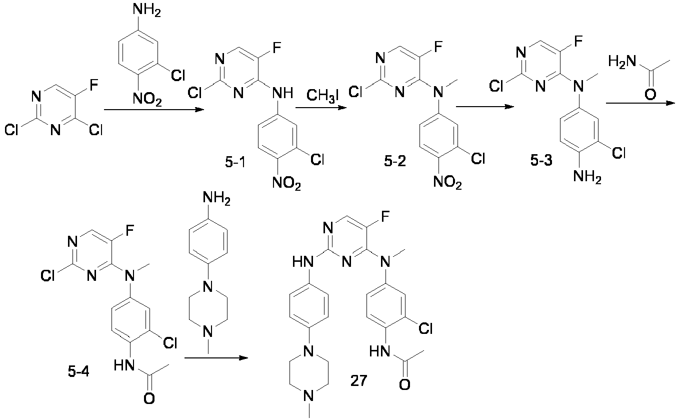

### Synthesis of an intermediate 5-1:

### 2-chloro-N-(3-chloro-4-nitrophenyl)-5-fluoropyrimidine-4-amine

2,4-dichloro-5-fluoropyrimidine (166.0 mg, 1.00 mmol), 3-chloro-4-nitroaniline (206.4 mg, 1.20 mmol), and anhydrous sodium carbonate (318.0 mg, 3.00 mmol) were fully dissolved in anhydrous ethanol (10 mL) to obtain a mixture, the mixture was condensed and refluxed at 80°C, and was distilled under reduced pressure, the reaction was completed after 2 h, the mixture was distilled under reduced pressure, extracted with ethyl acetate and concentrated under reduced pressure, and then separated and purified by column chromatography to obtain a yellow solid Compound 5-1, with a yield of 93.4%.

### Synthesis of an intermediate 5-2:

### 2-chloro-N-(3-chloro-4-nitrophenyl)-5-fluoro-N-methylpyrimidine-4-amine

Compound 5-1 (271.8 mg, 0.90 mmol) and cesium carbonate (879.7 mg, 2.70 mmol) were dissolved in anhydrous DMF (5 mL), stirred thoroughly at room temperature, and iodomethane (184.5 mg, 1.30 mmol) was added dropwise to the system for reaction, the reaction was completed after 1 h to obtain a mixture, ice water was poured into the mixture, a great amount of solid was precipitated, the mixture was allowed to stand, filtered and washed with water, and then dried to obtain a yellow solid Compound 5-2, with a yield of 91.5%.

### Synthesis of an intermediate 5-3:

### 3-chloro-N¹-(2-chloro-5-fluoropyrimidine-4-yl)-N¹-methylbenzene-1,4-diamine

Compound 5-2 (252.8 mg, 0.80 mmol) and anhydrous ammonium chloride (189.0 mg, 3.50 mmol) were fully dissolved in a mixed solution (EtOH : H₂O=3 : 1, 8 mL) to obtain a mixture, the mixture was condensed and refluxed at 60°C for 30 min, followed by the addition of iron powder (196.0 g, 3.50 mmol) and heating to 80°C for reaction, the reaction was completed after 3 h, the mixture was then distilled under reduced pressure, filtered and extracted with ethyl acetate, and concentrated under reduced pressure, and then separated and purified by column chromatography to obtain a solid Compound 5-3, with a yield of 95.1%.

### Synthesis of an intermediate 5-4:

### N-(2-chloro-4-((2-chloro-5-fluoropyrimidine-4-yl)(methyl)amino)phenyl)acetamide

Compound 5-3 (200.2 mg, 0.70 mmol) was fully dissolved in anhydrous tetrahydrofuran (5 mL), and anhydrous sodium carbonate (254.4 mg, 2.40 mmol) was added and stirred for 5 min at 0°C to obtain a mixture, acryloyl chloride (100 µL, 1.20 mmol) was added dropwise to the mixture for reaction, the reaction was completed after 1 h, the mixture was then quenched with NH₄Cl solution, distilled under reduced pressure, extracted with ethyl acetate and concentrated under reduced pressure, and then separated and purified by column chromatography to obtain a light yellow solid Compound 5-4, with a yield of 73.0%.

### Synthesis of Compound 27:

### N-(2-chloro-4-((5-fluoro-2-((4-(4-methylpiperazine-1-yl)phenyl)amino)pyrimidine-4-yl)(methyl) amino)phenyl)acetamide

Compound 5-4 (142.0 mg, 0.50 mmol) and 4-(4-ethylpiperazine-1-yl)aniline (114.8 mg, 0.60 mmol) were fully dissolved in 2-butanol (5 mL), and trifluoroacetic acid (75 µL, 1.00 mmol) was added dropwise to obtain a mixture, the mixture was condensed and refluxed at 100 °C for reaction, the reaction was completed after 8 h, the mixture was distilled under reduced pressure, extracted with ethyl acetate and concentrated under reduced pressure, and then separated and purified by column chromatography to obtain a yellow solid Compound 27, with a yield of 20.6%.

### Example 6: Preparation of

### N-(2-chloro-4-((5-chloro-2-((4-((3R,5S)-3,5-dimethylpiperazine-1-yl)-3-methoxyphenyl)amino) pyrimidine-4-yl) )amino)phenyl)acrylamide (Compound 46)

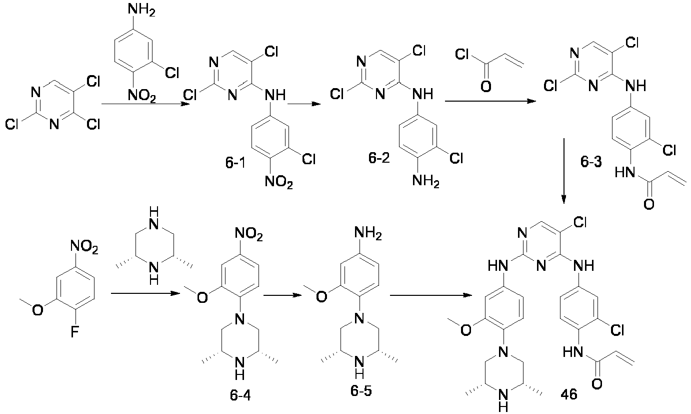

### Synthesis of an intermediate 6-1:

### 2,5-dichloro-N-(3-chloro-4-nitrophenyl)pyrimidine-4-amine

2,4,5-trichloropyrimidine (167.0 mg, 1.00 mmol), 3-chloro-4-nitroaniline (206.4 mg, 1.20 mmol), and anhydrous sodium carbonate (318.0 mg, 3.00 mmol) was fully dissolved in anhydrous ethanol (10 mL), and stirred at room temperature for reaction, the reaction was completed after 2 h to obtain a mixture, the mixture was then distilled under reduced pressure, and extracted with ethyl acetate, organic layers thereof were combined, dried, and concentrated under reduced pressure, and then separated and purified by column chromatography to obtain a yellow solid Compound 6-1, with a yield of 92.3%.

### Synthesis of an intermediate 6-2:

### 3-chloro-N¹-(2,5-dichloropyrimidine-4-yl)benzene-1,4-diamine

Compound 6-1 (287.5 mg, 0.90 mmol) and anhydrous ammonium chloride (189.0 mg, 3.50 mmol) were fully dissolved in a mixed solution (EtOH: H₂O=3 : 1, 8 mL) to obtain a mixture, the mixture was condensed and refluxed at 60°C for 30 min, followed by the addition of iron powder (196.0 g, 3.50 mmol) and heating to 80°C for reaction, the reaction was completed after 3 h, the mixture was then distilled under reduced pressure, filtered and extracted with ethyl acetate, and concentrated under reduced pressure, and then separated and purified by column chromatography to obtain a yellow solid Compound 6-2, with a yield of 94.2%.

### Synthesis of an intermediate 6-3:

### N-(2-chloro-4-((2,5-dichloropyrimidine-4-yl)amino)phenyl)acrylamide

Compound 6-2 (202.6 mg, 0.70 mmol) was fully dissolved in anhydrous tetrahydrofuran (5 mL), and anhydrous sodium carbonate (254.4 mg, 2.40 mmol) was added and stirred for 5 min at 0°C to obtain a mixture, acryloyl chloride (100 µL, 1.20 mmol) was added dropwise to the mixture for reaction, the reaction was completed after 1 h, the mixture was then quenched with NH₄Cl solution, distilled under reduced pressure, extracted with ethyl acetate and concentrated under reduced pressure, and then separated and purified by column chromatography to obtain a solid Compound 6-3, with a yield of 72.0%.

### Synthesis of an intermediate 6-4:

### (3R,5S)-1-(2-methoxy-4-nitrophenyl)-3,5-dimethylpiperazine

1-fluoro-2-methoxy-4-nitrobenzene (171.1 mg, 1.00 mmol), (2R,6S)-2,6-dimethylpiperazine (114.2 mg, 1.00 mmol), and anhydrous potassium carbonate (414.6 mg, 3.00 mmol) were dissolved in DMSO (5 mL) to obtain a mixture, the mixture was condensed and refluxed at 80 °C for reaction, the reaction was completed after 2 h, ice water was poured into the mixture to precipitate a solid, and the mixture was then allowed to stand, filtered and washed with water, and then dried to obtain a yellow solid Compound 6-4, with a yield of 63.1%.

### Synthesis of an intermediate 6-5:

### 4-((3R,55)-3,5-dimethylpiperazine-1-yl)-3-methoxyaniline

Compound 6-4 (132.6 mg, 0.5 mmol) was dissolved in methanol (5 mL), and 13 mg of Pd/C was added under an atmosphere of hydrogen (H₂) protection for reaction, the reaction was completed after 1 h to obtain a mixture, the mixture was filtered, distilled under reduced pressure, extracted with ethyl acetate and concentrated under reduced pressure, and then separated and purified by column chromatography to obtain a solid Compound 6-5, with a yield of 89.2%.

### Synthesis of Compound 46:

### N-(2-chloro-4-((5-chloro-2-((4-((3R,5S)-3,5-dimethylpiperazine-1-yl)-3-methoxyphenyl)amino) pyrimidine-4-yl) )amino)phenyl)acrylamide

Compound 6-3 (171.0 mg, 0.5 mmol) and Compound 6-5 (117.6 mg, 0.5 mmol) were fully dissolved in 2-butanol (5 mL), and trifluoroacetic acid (75 µL, 1.00 mmol) was added dropwise to obtain a mixture, the mixture was condensed and refluxed at 120 °C for reaction, the reaction was completed after 8 h, the mixture was distilled under reduced pressure, extracted with ethyl acetate and concentrated under reduced pressure, and then separated and purified by column chromatography to obtain a white solid Compound 46, with a yield of 36.9%.

### Example 7:

### N-(2-chloro-4-((5-chloro-2-((5-(4-methylpiperazine-1-yl)pyridine-2-yl)amino)pyrimidine-4-yl)a mino)phenyl)acrylamide (Compound 45)

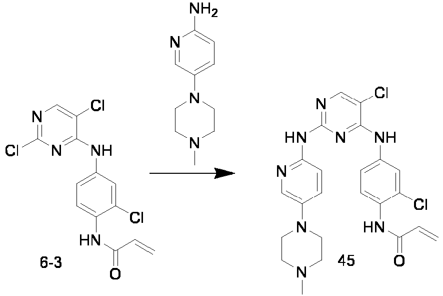

Compound 6-3 (171.0 mg, 0.50 mmol), 5-(4-methylpiperazine-1-yl)pyridine-2-amine (115.4 mg, 0.60 mmol), Pd₂(dba)₃( 22.9 mg, 0.025 mmol), BINAP (31.1 mg, 0.05 mmol), and NaOtBu (72.1 mg, 0.75 mmol) were dissolved in anhydrous 1,4-dioxane (5 mL) to obtain a mixture, the mixture was refluxed at 110°C under an atmosphere of nitrogen (N₂) protection, the mixture was distilled under reduced pressure, extracted with ethyl acetate and concentrated under reduced pressure after the completion of reaction, and then separated and purified by column chromatography to obtain a white solid Compound 45, with a yield of 11.8%.

The following 2,4,5-trisubstituted pyrimidine compounds were synthesized by modifying the starting materials according to synthetic routes and methods similar to those described in Examples 1-7.

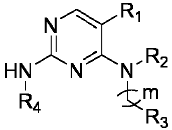

| S/N | R₁ | R₂ | m | R₃ | R₄ |
|---|---|---|---|---|---|
| 1 | -F | -H | 0 | 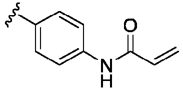 | 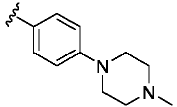 |
| 2 | -Cl | -H | 0 | 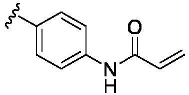 | 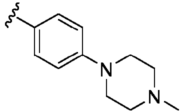 |
| 3 | -CH₃ | -H | 0 | 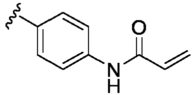 | 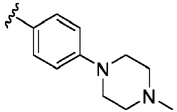 |
| 4 | -OCH₃ | -H | 0 | 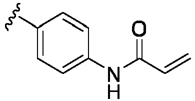 | 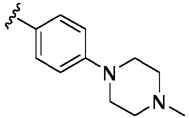 |
| 5 | -F | -H | 0 | 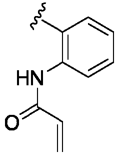 | 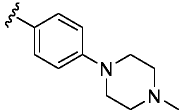 |
| 6 | -CH₃ | -H | 0 | 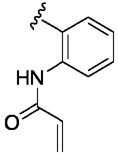 | 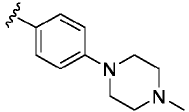 |
| 7 | -OCH₃ | -H | 0 | 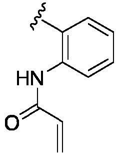 | 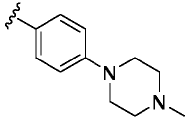 |
| 8 | -CF₃ | -H | 0 | 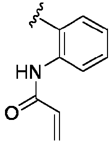 | 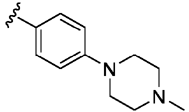 |
| 9 | -Cl | -H | 0 | 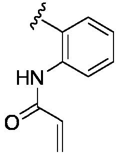 | 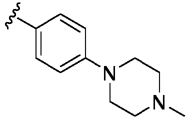 |
| 10 | -F | -H | 0 | 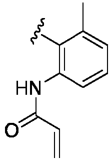 | 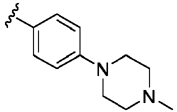 |
| 11 | -Cl | -H | 0 | 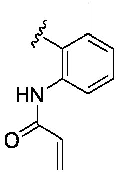 | 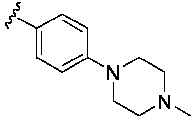 |
| 12 | -CH₃ | -H | 0 | 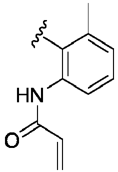 | 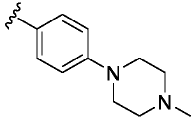 |
| 13 | -OCH₃ | -H | 0 | 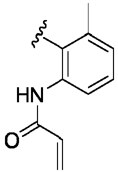 | 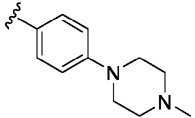 |
| 14 | -CF₃ | -H | 0 | 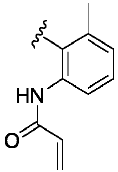 | 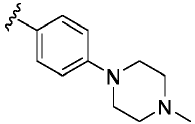 |
| 15 | -F | -H | 0 | 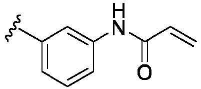 | 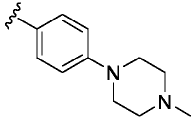 |
| 16 | -CH₃ | -H | 0 | 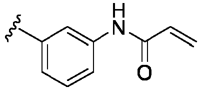 | 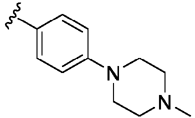 |
| 17 | -F | -H | 2 | 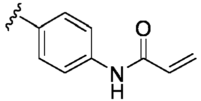 | 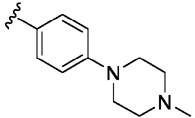 |
| 18 | -F | -H | 1 | 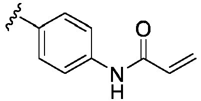 | 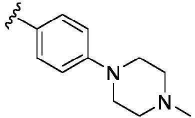 |
| 19 | -F | -H | 0 | 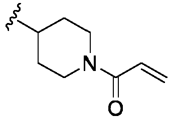 | 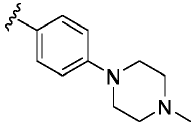 |
| 20 | -F | -H | 0 | 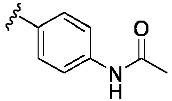 | 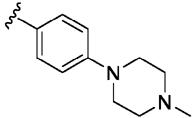 |
| 21 | -F | -H | 0 | 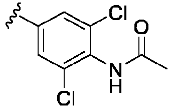 | 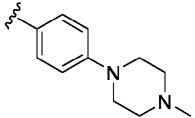 |
| 22 | -Cl | -H | 0 | 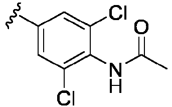 | 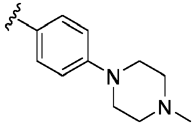 |
| 23 | -F | -H | 0 | 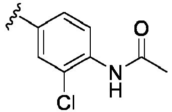 | 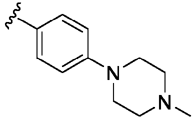 |
| 24 | -F | -H | 0 | 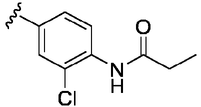 | 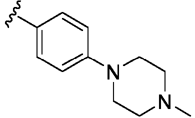 |
| 25 | -F | -H | 0 | 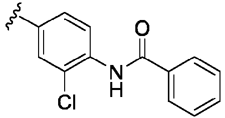 | 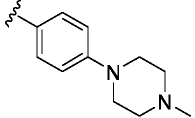 |
| 26 | -Cl | -H | 0 | 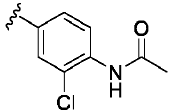 | 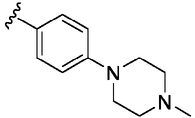 |
| 27 | -F | -CH₃ | 0 | 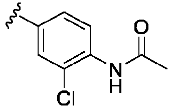 | 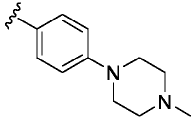 |
| 28 | -F | -C₂H₅ | 0 | 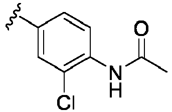 | 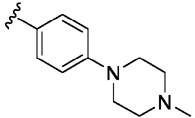 |
| 29 | -F | -H | 0 | 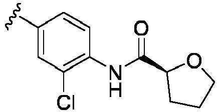 | 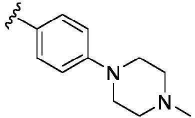 |
| 30 | -Cl | -H | 0 | 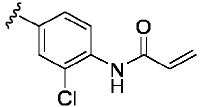 | 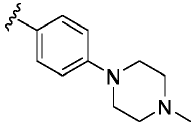 |
| 31 | -Cl | -H | 0 | 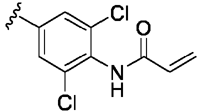 | 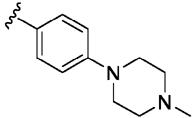 |
| 32 | -Cl | -H | 0 | 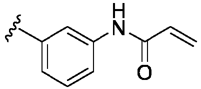 | 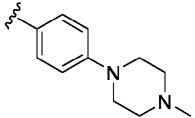 |
| 33 | -Cl | -H | 2 | 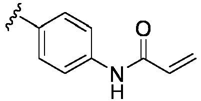 | 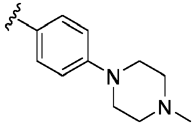 |
| 34 | -Cl | -H | 0 | 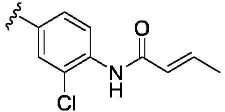 | 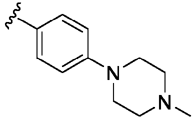 |
| 35 | -Cl | -H | 0 | 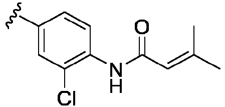 | 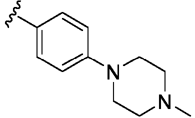 |
| 36 | -Cl | -H | 0 | 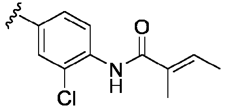 | 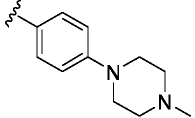 |
| 37 | -Cl | -H | 0 | 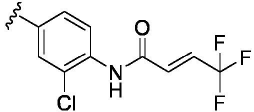 | 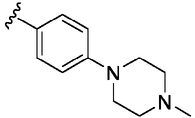 |
| 38 | -Cl | -H | 0 | 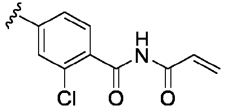 | 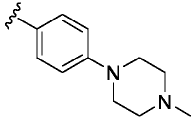 |
| 39 | -Cl | -H | 0 | 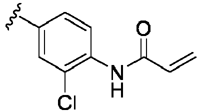 | 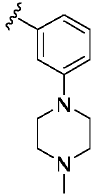 |
| 40 | -Cl | -H | 0 | 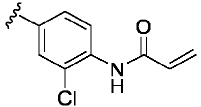 | 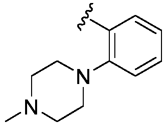 |
| 41 | -Cl | -H | 0 | 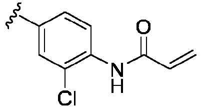 | 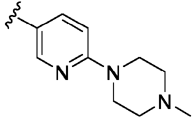 |
| 42 | -Cl | -H | 0 | 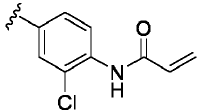 | 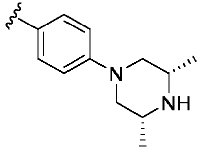 |
| 43 | -Cl | -H | 0 | 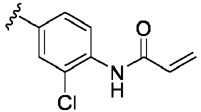 | 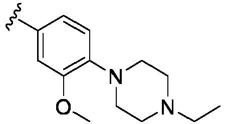 |
| 44 | -Cl | -H | 0 | 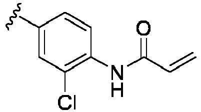 | 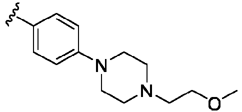 |
| 45 | -Cl | -H | 0 | 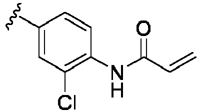 | 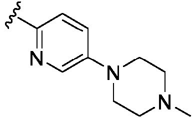 |
| 46 | -Cl | -H | 0 | 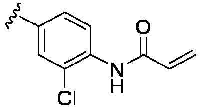 | 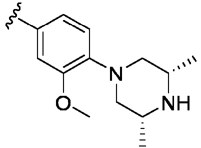 |
| 47 | -Cl | -H | 0 | 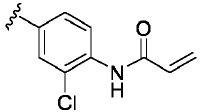 | 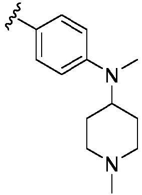 |
| 48 | -Cl | -H | 0 | 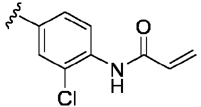 | 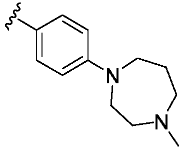 |
| 49 | -Cl | -H | 0 | 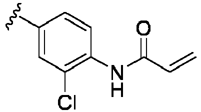 | 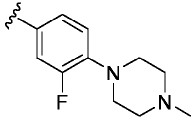 |
| 50 | -Cl | -H | 0 | 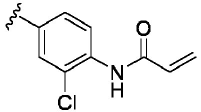 | 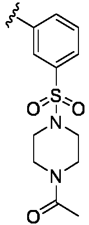 |
| 51 | -Cl | -H | 0 | 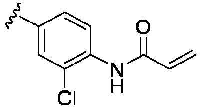 | 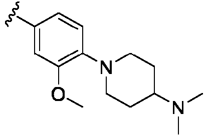 |
| 52 | -Cl | -H | 0 | 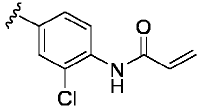 | 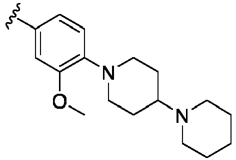 |

### Example 8: Determination of the activity of compounds on FGFR1 kinase

The inhibitory activity of the compounds on FGFR1 kinase was determined using a LANCE ULTRA Assay. The FGFR1 kinase was purchased from CARNA. Specific method was as follows: the compounds under test, adenosine triphosphate (ATP), specific substrate, and FGFR1 kinase were diluted with kinase diluent. A kinase reaction mixture contained FGFR1, ATP, substrate, HEPES (pH = 7.5), MgCl2, EGTA, and Tween-20. A group without any test compound served as a 100% phosphorylation control, and a group where EDTA was added immediately after the addition of FGFR1 kinase served as a 0% phosphorylation control. The kinase reaction mixture was incubated at room temperature for 1 h, and EDTA was added to terminate the reaction for 5 min, a specific antibody was then added, and the mixture was incubated at room temperature for another 1 h, and excitation light was detected using a PerkinElmer Envision Kinase Reader. According to a signal value obtained from the kinase reader, an inhibition rate was calculated using the following formula or an IC₅₀ value was determined using GraphPad, and results were shown as follows:

| Compound No. | In vitro FGFR1 kinase inhibition rate (%) (Concentration in 1000 nM) |
|---|---|
| 2 | 92.6 |
| 4 | 45.49 |
| 5 | 65.58 |
| 6 | 49.49 |
| 8 | 23.31 |
| 9 | 94.91 |
| 10 | 1.23 |

| S/N | In vitro FGFR1 kinase inhibition rate (%) (Concentration in 100 nM) | S/N | In vitro FGFR1 kinase inhibition rate (%) (Concentration in 100 nM) |
|---|---|---|---|
| 1 | 33.9 | 28 | 22.2 |
| 2 | 72.9 | 29 | 24.1 |
| 3 | 8.4 | 30 | 73.3 |
| 7 | 24.4 | 31 | 61.2 |
| 11 | 1.4 | 32 | 54.6 |
| 12 | -1.1 | 33 | 66.4 |
| 13 | -0.1 | 38 | 50.29 |
| 14 | 11.4 | 39 | 73.38 |
| 15 | 37.4 | 40 | 24.99 |
| 16 | 54.6 | 41 | 91.64 |
| 17 | 43.0 | 42 | 63.78 |
| 18 | 5.5 | 43 | 79.33 |
| 19 | 1.7 | 44 | 47.07 |
| 20 | 24.2 | 45 | 15.25 |
| 21 | 33.7 | 46 | 78.31 |
| 22 | 60.1 | 47 | 52.33 |
| 23 | 36.5 | 48 | 53.57 |
| 24 | 32.8 | 49 | 81.76 |
| 25 | 27.8 | 50 | 32.87 |
| 26 | 57.0 | 51 | 75.11 |
| 27 | 11.4 | 52 | 71.86 |

| Compound No. | In vitro FGFR1 kinase IC₅₀ value (nM) |
|---|---|
| 30 | 32.6 |
| 31 | 73.9 |
| 32 | 85.0 |
| 33 | 60.7 |
| 35 | 92.8 |
| 36 | 34.3 |
| 37 | 133.5 |
| 41 | 55.0 |
| 46 | 41.05 |

Experimental results showed that all the compounds under test exhibited inhibitory activity against FGFR1 kinase in vitro.

### Example 9: Inhibitory activity of active compounds on wild-type and mutant FGFR1

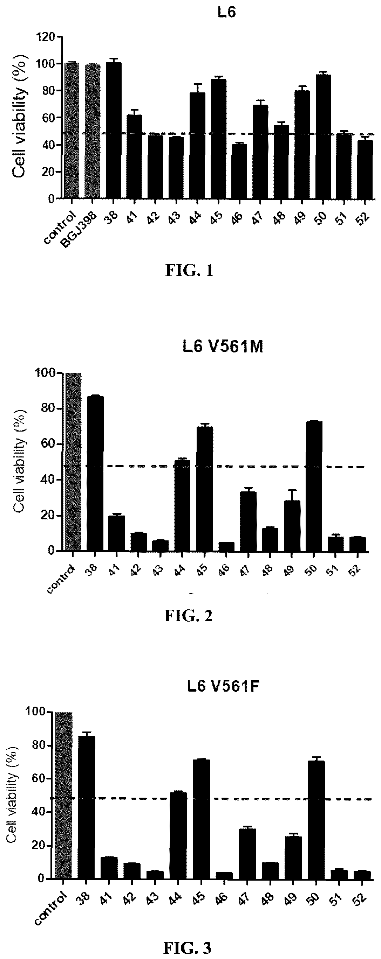

According to the method of Example 8, the inhibitory activity of some compounds on wild-type FGFR1 and FGFR1 mutants V561M and V561F was tested. Test results were shown in FIGs. 1-3.

All the compounds under test in the above examples demonstrated similar or better inhibitory activity against both the wild-type FGFR1 and the FGFR1 mutants V561M and V561F compared with BGJ398.

### Example 10: Effect of active compounds on proliferation of MRC-5, H1581, and A549 cells

The effect of active compounds on cell proliferation was measured using an MTT assay. An experimental method was described as follows: the compounds under test were first dissolved in DMSO to prepare a stock solution, which was then diluted in gradient with a culture medium of the corresponding cells to prepare test samples, with a final concentration range of the compounds up to 20 µM. Cells in a logarithmic growth phase were seeded into 96-well cell culture plates at an appropriate density, and cultured overnight under corresponding culture conditions, and samples of the compound under test were added and continuously cultured for 72 h. After the culture was completed, an appropriate volume of MTT detection solution was added to each well, and incubated at 37°C for 1-4 h, the culture medium was then carefully discarded, and 100 µL of DMSO was added to each well to dissolve crystals formed at a bottom of the well, the plates were placed on a micro-oscillator and shaken at room temperature in a dark for 10 min until the crystals were fully dissolved, an absorbance of each well at 490 nm was measured using a microplate reader, and an inhibition rate or IC₅₀ value of each compound on lung cancer cells was calculated.

| Compound | IC₅₀ (µM) | | |
|---|---|---|---|
| | MRC-5 | H1581 | A549 |
| 46 | 0.84 ± 0.07 | 0.01796 ± 0.00178 | 0.38 ± 0.10 |
| BGJ398 | 143.55 | 0.08673 ± 0.00151 | 10.36 ± 0.39 |

The preferred compounds of the present disclosure exhibit significant inhibitory effects on the proliferation of FGFR1-abnormal lung cancer cells H1581 and A549, demonstrating better cell proliferation inhibition activity than the positive control drug BGJ398.

**Example 11:** Determination of properties, yields, hydrogen spectra, carbon spectra and mass spectra of Compounds 1-52 were measured. Characterization data of some compounds were as follows:
N-(4-((5-fluoro-2-((4-(4-methylpiperazine-1-yl)phenyl)amino)pyrimidine-4-yl)amino)p henyl)acrylamide (1), gray-green solid with a yield of 23.1%. ¹H NMR (400 MHz, DMSO-*d₆*) δ 10.18 (s, 1H), 9.27 (s, 1H), 8.92 (s, 1H), 8.03 (d, *J* = 3.7 Hz, 1H), 7.76 (d, *J* = 8.8 Hz, 2H), 7.65 (d, *J* = 8.8 Hz, 2H), 7.49 (d, *J* = 8.8 Hz, 2H), 6.84 (d, *J* = 8.9 Hz, 2H), 6.47 (dd, *J* = 16.9, 10.1 Hz, 1H), 6.33 - 6.22 (m, 1H), 5.76 (dd, *J* = 10.1, 1.6 Hz, 1H), 3.13-2.97 (m, 4H), 2.50 - 2.41 (m, 4H), 2.24 (s, 3H).¹³C NMR (101 MHz, DMSO) δ 163.41, 156.34, 150.17, 150.06, 146.41, 135.22, 134.83, 133.63, 132.48, 130.15, 127.00, 122.22, 120.74, 120.04, 116.32, 55.20, 49.47, 46.23, 40.63.HRMS (ESI) m/z: calcd for C₂₄H₂₆FN₇O⁺, 448.2261; found, 448.2261. HPLC purity: 99.8%.
N-(2-((5-chloro-2-((4-(4-methylpiperazine-1-yl)phenyl)amino)pyrimidine-4-yl)amino)-3-methylphenyl)acrylamide (11), off-white solid with a yield of 16.7%. ¹H NMR (400 MHz, DMSO-d₆) δ 9.91 (s, 1H), 8.99 (s, 1H), 8.24 (s, 1H), 8.04 (s, 1H), 7.54 (d, *J =* 7.8 Hz, 1H), 7.32 (t, *J =* 7.8 Hz, 1H), 7.28 - 7.19 (m, 3H), 6.61 (d*, J =* 8.7 Hz, 2H), 6.54 (dd, *J* = 17.0, 10.2 Hz, 1H), 6.29 (dd, *J* = 17.0, 1.7 Hz, 1H), 5.82 - 5.69 (m, 1H), 3.07 - 2.87 (m, 4H), 2.49 - 2.41 (m, 4H), 2.21 (d, *J* = 19.1 Hz, 6H).¹³C NMR (101 MHz, DMSO) δ 164.45, 158.39, 157.23, 154.49, 146.03, 137.75, 134.89, 133.48, 131.88, 130.96, 130.16, 127.85, 127.72, 127.17, 121.88, 119.75, 116.14, 55.14, 49.37, 46.19, 19.05.HRMS (ESI) m/z: calcd for C₂₅H₂₈ClN₇O⁺, 478.2122; found, 478.2115.HPLC purity: 99.6%.
1-(4-((5-fluoro-2-((4-(4-methylpiperazine-1-yl)phenyl)amino)pyrimidine-4-yl)amino)pi peridine-1-yl)prop-2-en-1-one (19), white solid with a yield of 27.3%. ¹H NMR (400 MHz, CDCl₃) δ 7.80 (d, *J* = 3.2 Hz, 1H), 7.45 (d, *J=* 8.9 Hz, 2H), 6.93 (d, *J* = 8.9 Hz, 2H), 6.84 (s, 1H), 6.63 (dd, *J* = 16.8, 10.6 Hz, 1H), 6.33 (dd, *J* = 16.8, 1.8 Hz, 1H), 5.74 (dd, *J* = 10.6, 1.8 Hz, 1H), 4.86 (d, *J* = 7.2 Hz, 1H), 4.67 (d*, J =* 12.2 Hz, 1H), 4.28 - 3.98 (m, 2H), 3.33 - 3.16 (m, 5H), 2.94 (t, *J* = 12.8 Hz, 1H), 2.75 - 2.64 (m, 4H), 2.43 (s, 3H), 2.20 (s, 4H).¹³C NMR (101 MHz, CDCl₃) δ 165.57, 156.17, 146.68, 142.30, 139.39, 139.19, 133.23, 128.01, 127.65, 120.70, 117.19, 55.00, 49.67, 47.90, 45.79, 31.80, 29.75.HRMS (ESI) m/z: calcd for C₂₃H₃₀FN₇O⁺, 440.2574; found, 440.2561.HPLC purity: 95.7%.
N-(2-chloro-4-((5-fluoro-2-((4-(4-methylpiperazine-1-yl)phenyl)amino)pyrimidine-4-yl) (methyl)amino)phenyl)acetamide (27), yellow-brown solid with a yield of 20.6%. ¹H NMR (400 MHz, Chloroform-d) δ 8.40 (d, *J* = 8.8 Hz, 1H), 7.83 (d, *J* = 5.4 Hz, 1H), 7.66 (s, 1H), 7.44 (d, *J* = 8.9 Hz, 2H), 7.26 (*d, J =* 1.9 Hz, 1H), 7.16 (dd, *J* = 8.8, 2.2 Hz, 1H), 7.00 (s, 1H), 6.92 (d, *J =* 8.9 Hz, 2H), 3.48 (s, 3H), 3.25 - 3.16 (m, 4H), 2.69 - 2.63 (m, 4H), 2.41 (s, 3H), 2.28 (s, 3H).¹³C NMR (101 MHz, CDCl₃) δ 168.26, 155.96, 151.68, 146.81, 143.47, 143.23, 140.54, 132.96, 132.78, 125.98, 124.89, 122.66, 121.94, 120.62, 117.11, 55.13, 49.84, 46.00, 40.11, 24.86.HRMS (ESI) m/z: calcd for C₂₄H₂₇ClFN₇O⁺, 484.2028; found, 484.2027.HPLC purity: 99.7%.
N-(4-(2-((5-chloro-2-((4-(4-methylpiperazine-1-yl)phenyl)amino)pyrimidine-4-yl)amin o)ethyl)phenyl)acrylamide (33), light yellow solid with a yield of 18.6%. ¹H NMR (400 MHz, DMSO-d₆) δ 10.13 (s, 1H), 8.97 (s, 1H), 7.90 (s, 1H), 7.65 (d*, J =* 8.1 Hz, 2H), 7.56 (d*, J =* 8.6 Hz, 2H), 7.22 (d, *J* = 8.0 Hz, 3H), 6.78 (d, *J* = 8.7 Hz, 2H), 6.46 (dd, *J =* 16.9, 10.1 Hz, 1H), 6.28 (d, *J* = 16.8 Hz, 1H), 5.77 (d, *J* = 9.8 Hz, 1H), 3.69 - 3.56 (m, 2H), 3.04 (s, 4H), 2.92 - 2.80 (m, 2H), 2.47 (s, 4H), 2.23 (s, 3H).¹³C NMR (101 MHz, DMSO) δ 163.49, 158.75, 157.79, 153.55, 146.25, 137.71, 135.00, 133.52, 132.45, 129.41, 127.16, 120.54, 119.84, 116.22, 103.17, 55.16, 49.34, 46.20, 42.31, 34.90.HRMS (ESI) m/z: calcd for C₂₆H₃₀ClN₇O⁺, 492.2279; found, 492.2271.HPLC purity: 95.9%.
N-acryloyl-2-chloro-4-((5-chloro-2-((4-(4-methylpiperazine-1-yl)phenyl)amino)pyrimid ine-4-yl)amino)benzamide (38), yellow oily liquid with a yield of 11.2%. ¹H-NMR (400 MHz, DMSO-d₆) δ 9.22 (s, 1H), 8.95 (s, 1H), 8.16 (s, 1H), 7.91 (s, 1H), 7.83 (s, 1H), 7.80 - 7.71 (m, 1H), 7.53 (s, 1H), 7.48 - 7.34 (m, 4H), 6.88 (d, *J* = 8.7 Hz, 3H), 3.08 (s, 4H), 2.50 (d, *J* = 5.6 Hz, 4H), 2.26 (s, 3H).¹³C-NMR (101 MHz, DMSO) δ 168.47, 158.50, 155.88, 155.77, 146.85, 141.29, 133.24, 132.60, 131.77, 130.29, 130.16, 129.31, 127.07, 122.81, 121.55, 120.60, 116.46, 103.83, 55.09, 49.20, 46.13.HPLC purity: 94.5%.
N-(2-chloro-4-((5-chloro-2-((6-(4-methylpiperazine-1-yl)pyridine-3-yl)amino)pyrimidin e-4-yl)amino)phenyl)acrylamide (41), purple solid with a yield of 20.0%. ¹H NMR (400 MHz, DMSO-d₆) δ 9.70 (s, 1H), 9.15 (s, 1H), 8.90 (s, 1H), 8.19 (s, 1H), 8.12 (s, 1H), 7.87 - 7.75 (m, 2H), 7.67 (s, 2H), 6.78 (d, *J* = 9.1 Hz, 1H), 6.63 (dd, *J =* 16.9, 10.3 Hz, 1H), 6.29 (dd, *J =* 17.0, 1.6 Hz, 1H), 5.85 - 5.73 (m, 1H), 3.44 - 3.39 (m, 4H), 2.46 - 2.35 (m, 4H), 2.23 (s, 3H).¹³C NMR (101 MHz, DMSO) δ 164.05, 158.78, 158.13, 156.17, 155.71, 155.50, 140.59, 137.43, 131.92, 131.59, 130.57, 128.28, 127.69, 127.04, 126.73, 123.36, 122.16, 107.34, 54.83, 46.19, 45.59.HRMS (ESI) m/z: calcd for C₂₄H₂₄Cl₂N₈O⁺, 499.1528; found, 499.1526.HPLC purity: 96.7%.
N-(2-chloro-4-((5-chloro-2-((4-((3R,5S)-3,5-dimethylpiperazine-1-yl)phenyl)amino)pyr imidine-4-yl)amino)phenyl)acrylamide (42), white sold with a yield of 19.5%. ¹H NMR (400 MHz, DMSO-d₆) δ 9.74 (s, 1H), 9.17 (s, 1H), 8.91 (s, 1H), 8.13 (s, 1H), 7.83 (s, 1H), 7.69 (s, 2H), 7.43 (d, *J* = 8.6 Hz, 2H), 6.86 (d, *J* = 9.0 Hz, 2H), 6.64 (dd, *J* = 16.9, 10.2 Hz, 1H), 6.30 (dd, *J =* 17.0, 1.6 Hz, 1H), 5.85 - 5.75 (m, 1H), 3.50 (d, *J* = 10.4 Hz, 4H), 2.19 (t, *J* = 11.0 Hz, 2H), 1.08 (d, *J =* 6.2 Hz, 7H).¹³C NMR (101 MHz, DMSO) δ 164.04, 158.47, 156.18, 155.49, 146.50, 137.45, 132.72, 131.99, 130.63, 127.61, 126.98, 126.66, 123.64, 122.64, 122.50, 121.35, 116.49, 55.63, 50.77, 19.08.HRMS (ESI) m/z: calcd for C₂₅H₂₇Cl₂N₇O⁺, 512.1732; found, 512.1731.HPLC purity: 94.6%.
N-(2-chloro-4-((5-chloro-2-((4-(4-ethylpiperazine-1-yl)-3-methoxyphenyl)amino)pyrim idine-4-yl)amino)phenyl)acrylamide (43), beige solid with a yield of 18.3%. ¹H NMR (400 MHz, DMSO-d₆) δ 9.71 (s, 1H), 9.22 (s, 1H), 8.92 (s, 1H), 8.16 (s, 1H), 7.92 (s, 1H), 7.70 (s, 2H), 7.25 - 7.18 (m, 1H), 7.15 (s, 1H), 6.80 (d, *J* = 8.6 Hz, 1H), 6.64 (dd, *J* = 17.0, 10.3 Hz, 1H), 6.30 (dd, *J* = 17.0, 1.9 Hz, 1H), 5.80 (dd, *J* = 10.2, 1.8 Hz, 1H), 3.64 (s, 3H), 2.93 (s, 4H), 2.55 - 2.52 (m, 4H), 2.45 - 2.33 (m, 2H), 1.04 (t, *J =* 7.1 Hz, 3H).¹³C NMR (101 MHz, DMSO) δ 164.03, 158.35, 156.04, 155.44, 152.36, 137.43, 136.33, 135.90, 132.05, 131.93, 130.57, 127.70, 126.97, 126.63, 123.26, 121.97, 118.38, 112.33, 105.19, 104.03, 55.59, 53.07, 52.17, 50.70, 12.31.HRMS (ESI) m/z: calcd for C₂₆H₂₉Cl₂N₇O⁺, 542.1838; found, 542.1837.HPLC purity: 99.0%.
N-(2-chloro-4-((5-chloro-2-((4-(4-(2-methoxyethyl) piperazine -1-yl)phenyl)amino)pyrimidine-4-yl)amino)phenyl)acrylamide (44), beige solid with a yield of 17.4%. ¹H NMR (400 MHz, DMSO-d₆) δ 9.71 (s, 1H), 9.16 (s, 1H), 8.89 (s, 1H), 8.12 (s, 1H), 7.87 (s, 1H), 7.70 (t, *J* = 10.7 Hz, 2H), 7.43 (d, *J* = 8.5 Hz, 2H), 6.85 (d, *J* = 8.8 Hz, 2H), 6.65 (dd, *J* = 16.7, 10.1 Hz, 1H), 6.30 (d, *J =* 16.9 Hz, 1H), 5.81 (d, *J =* 10.2 Hz, 1H), 3.49 (t, *J =* 5.6 Hz, 2H), 3.27 (s, 4H), 3.06 (s, 4H), 2.58 (s, 5H).¹³C NMR (101 MHz, DMSO) δ 164.05, 158.45, 156.18, 155.48, 146.71, 137.45, 132.77, 131.94, 130.63, 127.69, 126.98, 126.70, 123.59, 122.43, 121.24, 116.38, 103.56, 70.28, 58.54, 57.47, 53.61, 49.32, 40.64, 40.43, 40.22, 40.01, 39.81, 39.60, 39.39.HRMS (ESI) m/z: calcd for C₂₆H₂₉Cl₂N₇O₂⁺, 542.1838; found, 542.1834.HPLC purity: 97.9%.
N-(2-chloro-4-((5-chloro-2-((5-(4-methylpiperazine-1-yl)pyridine-2-yl)amino)pyrimidin e-4-yl)amino)phenyl)acrylamide (45), off-white solid with a yield of 10.6%. ¹H NMR (400 MHz, DMSO-d₆) δ 9.76 (s, 1H), 9.49 (s, 1H), 9.01 (s, 1H), 8.20 (s, 1H), 7.99 (s, 1H), 7.89 (s, 1H), 7.78 (d, *J* = 9.1 Hz, 1H), 7.75 - 7.64 (m, 2H), 7.43 - 7.31 (m, 1H), 6.64 (dd, *J* = 16.5, 10.3 Hz, 1H), 6.30 (d, *J =* 17.1 Hz, 1H), 5.81 (d, *J =* 10.2 Hz, 1H), 3.15 (s, 4H), 2.57 (s, 4H), 2.30 (s, 3H).¹³C NMR (101 MHz, DMSO) δ 164.09, 157.49, 156.27, 155.42, 146.00, 142.84, 137.39, 135.70, 131.89, 130.79, 127.72, 127.16, 126.89, 125.85, 123.85, 122.73, 114.37, 104.69, 54.69, 48.62, 45.84.HRMS (ESI) m/z: calcd for C₂₄H₂₄Cl₂N₈O⁺, 499.1528; found, 499.1527.HPLC purity: 94.8%.
N-(2-chloro-4-((5-chloro-2-((4-((3R,5S)-3,5-dimethylpiperazine-1-yl)-3-methoxyphenyl )amino)pyrimidine-4-yl) )amino)phenyl)acrylamide (46), off-white solid with a yield of 19.9%.¹H NMR (400 MHz, DMSO-d₆) δ 9.71 (s, 1H), 9.21 (s, 1H), 8.91 (s, 1H), 8.16 (s, 1H), 7.89 (s, 1H), 7.70 (s, 2H), 7.26 - 7.08 (m, 2H), 6.78 (d, *J* = 8.6 Hz, 1H), 6.64 (dd, *J =* 17.0, 10.2 Hz, 1H), 6.30 (dd, *J* = 17.0, 1.8 Hz, 1H), 5.80 (dd, *J =* 10.2, 1.8 Hz, 1H), 3.64 (s, 3H), 3.15 (d, *J* = 9.6 Hz, 2H), 2.99 - 2.87 (m, 2H), 2.09 (t, *J =* 10.5 Hz, 2H), 0.99 (d, *J* = 6.3 Hz, 7H).¹³C NMR (101 MHz, DMSO) δ 164.02, 158.37, 156.04, 155.45, 152.39, 137.44, 136.54, 135.74, 131.94, 130.56, 127.68, 126.96, 126.62, 123.25, 122.00, 118.57, 112.46, 105.34, 104.00, 57.95, 55.64, 50.85, 19.78.HRMS (ESI) m/z: calcd for C₂₆H₂₉Cl₂N₇O₂⁺, 542.1838; found, 542.1832.HPLC purity: 96.8%.
N-(2-chloro-4-((5-chloro-2-((4-((3R,5S)-3,5-dimethylpiperazine-1-yl)-3-methoxyphenyl )amino)pyrimidine-4-yl) )amino)phenyl)acrylamide (47), brown solid with a yield of 17.3%. ¹H NMR (400 MHz, Chloroform-d) δ 8.41 (d, *J =* 8.1 Hz, 1H), 8.02 (s, 1H), 7.90 (d, *J =* 2.1 Hz, 1H), 7.70 (s, 1H), 7.33 (d, *J* = 8.8 Hz, 2H), 7.02 (s, 1H), 6.89 (s, 1H), 6.82 (d, *J* = 8.9 Hz, 2H), 6.44 (d, *J* = 16.8 Hz, 1H), 6.32 (dd, *J =* 16.9, 10.1 Hz, 1H), 5.83 (d, *J =* 10.1 Hz, 1H), 5.30 (s, 1H), 3.59 (t*, J =* 11.6 Hz, 1H), 3.48 (s, 2H), 3.11 (d, *J =* 11.3 Hz, 2H), 2.78 (s, 3H), 2.43 (s, 3H), 2.02 (d, *J =* 15.5 Hz, 4H).¹³C NMR (101 MHz, CDCl₃) δ 163.92, 163.27, 158.70, 155.28, 154.61, 146.86, 131.20, 130.33, 128.20, 122.90, 121.87, 121.22, 120.28, 120.23, 115.59, 115.19, 104.16, 56.54, 55.29, 45.45, 29.75, 29.37, 28.14.HRMS (ESI) m/z: calcd for C₂₆H₂₉Cl₂N₇O⁺, 526.1889; found, 526.1884.HPLC purity: 94.2%.
N-(2-chloro-4-((5-chloro-2-((4-(4-methyl-1,4-diazepine-1-yl)phenyl)amino)pyrimidine-4-yl)amino)phenyl)acrylamide (48), yellow solid with a yield of 16.5%. ¹H NMR (400 MHz, DMSO-d₆) δ 9.71 (s, 1H), 9.02 (s, 1H), 8.85 (s, 1H), 8.09 (s, 1H), 7.86 (s, 1H), 7.69 (s, 2H), 7.34 (d, *J =* 8.2 Hz, 2H), 6.62 (d, *J* = 8.7 Hz, 3H), 6.29 (d, *J =* 17.0 Hz, 1H), 5.85 - 5.74 (m, 1H), 3.48 (d, *J* = 19.6 Hz, 6H), 2.68 (s, 2H), 2.32 (s, 3H), 1.98 - 1.85 (m, 2H).¹³C NMR (101 MHz, DMSO) δ 164.04, 158.68, 156.09, 155.50, 144.96, 137.52, 131.97, 130.49, 129.53, 127.63, 126.96, 126.63, 123.36, 122.35, 122.23, 111.97, 103.13, 57.60, 56.66, 48.20, 47.98, 46.07, 26.92.HRMS (ESI) m/z: calcd for C₂₅H₂₇Cl₂N₇O⁺, 512.1732; found, 512.1733.HPLC purity: 82.0%.
N-(2-chloro-4-((5-chloro-2-((3-fluoro-4-(4-methylpiperazine-1-yl)phenyl)amino)pyrimi dine-4-yl)amino)phenyl)acrylamide (49), white solid with a yield of 20.6%. ¹H NMR (400 MHz, DMSO-d₆) δ 9.73 (s, 1H), 9.42 (s, 1H), 9.00 (s, 1H), 8.20 (s, 1H), 7.87 (d, *J* = 1.9 Hz, 1H), 7.81 - 7.63 (m, 2H), 7.48 (d, *J =* 15.1 Hz, 1H), 7.34 (d, *J =* 8.7 Hz, 1H), 6.95 (t*, J =* 9.4 Hz, 1H), 6.66 (dd, *J =* 16.9, 10.2 Hz, 1H), 6.37 - 6.26 (m, 1H), 5.82 (d, *J =* 11.9 Hz, 1H), 2.96 (s, 4H), 2.59 - 2.51 (m, 4H), 2.26 (s, 3H).¹³C NMR (101 MHz, DMSO) δ 164.08, 158.01, 156.25, 155.40, 153.92, 137.25, 135.97, 134.32, 131.92, 130.86, 127.74, 126.65, 123.75, 122.44, 119.71, 115.66, 108.00, 107.75, 104.51, 55.12, 50.66, 46.03.HRMS (ESI) m/z: calcd for C₂₄H₂₄Cl₂FN₇O⁺, 516.1482; found, 516.1481.HPLC purity: 99.1%.
N-(4-((2-((3-((4-acetylpiperazine-1-yl)sulfonyl)phenyl)amino)-5-chloropyrimidine-4-yl) amino)-2-chlorophenyl)acrylamide (50), white solid with a yield of 14.8%. ¹H NMR (400 MHz, DMSO-d₆) δ 9.89 - 9.55 (m, 2H), 9.08 (s, 1H), 8.36 - 8.07 (m, 2H), 8.06 - 7.79 (m, 2H), 7.70 (d, *J* = 27.8 Hz, 2H), 7.50 (t, *J* = 7.8 Hz, 1H), 7.25 (d, *J* = 7.4 Hz, 1H), 6.63 (dd, *J* = 16.3, 10.4 Hz, 1H), 6.31 (d, *J* = 16.9 Hz, 1H), 5.81 (d, *J =* 10.2 Hz, 1H), 3.52 (s, 4H), 2.90 (d, *J =* 12.1 Hz, 4H), 1.94 (s, 3H).¹³C NMR (101 MHz, DMSO) δ 168.89, 164.13, 157.78, 156.40, 155.23, 141.89, 137.20, 135.53, 131.87, 130.98, 130.08, 127.84, 126.84, 123.97, 123.42, 122.49, 120.24, 117.90, 105.74, 60.28, 46.31, 45.42, 21.57, 14.59.HRMS (ESI) m/z: calcd for C₂₅H₂₅Cl₂N₇O₄S⁺, 590.1144; found, 590.1140.HPLC purity: 99.7%.
N-(2-chloro-4-((5-chloro-2-((4-(4-(dimethylamino)piperidine-1-yl)-3-methoxyphenyl)a mino)pyrimidine-4-yl)amino)phenyl)acrylamide (51), dark brown solid with a yield of 19.3%. ¹H NMR (400 MHz, DMSO-d₆) δ 9.74 (s, 1H), 9.22 (s, 1H), 8.93 (s, 1H), 8.17 (s, 1H), 7.91 (s, 1H), 7.70 (s, 2H), 7.26 - 7.12 (m, 2H), 6.81 (d, *J* = 8.5 Hz, 1H), 6.66 (dd, *J =* 16.7, 10.1 Hz, 1H), 6.31 (d, *J* = 17.1 Hz, 1H), 5.87 - 5.75 (m, 1H), 3.65 (s, 3H), 3.35 (d*, J =* 11.0 Hz, 4H), 2.35 (s, 7H), 1.88 (d, *J =* 10.5 Hz, 2H), 1.58 (q, *J =* 11.3, 10.8 Hz, 2H).¹³C NMR (101 MHz, DMSO) δ 164.02, 158.35, 156.05, 155.43, 152.40, 137.44, 136.59, 135.89, 131.94, 130.58, 127.67, 127.00, 126.67, 123.28, 122.00, 118.67, 112.26, 105.06, 104.00, 62.31, 55.57, 50.59, 41.38, 28.40.HRMS (ESI) m/z: calcd for C₂₇H₃₁Cl₂N₇O₂⁺, 556.1995; found, 556.1988.HPLC purity: 94.7%.
N-(4-((2-((4-([1,4'-bipiperidine]-1'-yl)-3-methoxyphenyl)amino)-5-chloropyrimidine-4-yl)amino)-2-chlorophenyl)acrylamide (52), light yellow solid with a yield of 17.7%. ¹H NMR (400 MHz, DMSO-d₆) δ 9.75 (s, 1H), 9.23 (s, 1H), 8.93 (s, 1H), 8.16 (s, 1H), 7.90 (s, 1H), 7.69 (s, 2H), 7.24 - 7.11 (m, 2H), 6.81 (d, *J =* 8.6 Hz, 1H), 6.65 (dd, *J* = 16.7, 10.3 Hz, 1H), 6.36 - 6.24 (m, 1H), 5.86 - 5.76 (m, 1H), 3.64 (s, 3H), 2.90 (s, 4H), 2.48 (s, 1H), 1.97 (q, *J =* 12.4, 10.5 Hz, 4H), 1.70 (s, 8H), 1.50 (s, 2H).¹³C NMR (101 MHz, DMSO) δ 164.05, 158.31, 156.06, 155.43, 152.39, 137.44, 136.19, 136.07, 131.92, 130.59, 127.73, 127.05, 126.69, 123.29, 122.00, 118.77, 118.70, 112.17, 104.96, 62.93, 55.58, 50.50, 49.77, 27.38, 24.52, 22.60.HRMS (ESI) m/z: calcd for C₃₀H₃₅Cl₂N₇O₂⁺, 596.2308; found, 596.2308.HPLC purity: 93.4%.

The above detailed description provides a specific explanation of feasible embodiments of the present disclosure. These embodiments are not intended to limit the scope of protection of the present disclosure. Any equivalent implementations or modifications that do not deviate from the present disclosure should be included within the scope of protection of the present disclosure.

In addition, those skilled in the art may also make various modifications, additions, and substitutions in other forms and details within the scope and spirit disclosed in the claims of the present disclosure. Naturally, all of these modifications, additions, and substitutions made in accordance with the spirit of the present disclosure should be included within the scope of protection claimed by the present disclosure.

## Claims

1. A 2,4,5-trisubstituted pyrimidine compound having a structure as represented by Formula (I) compound, an isotope labeled compound, or an optical isomer, a geometric isomer, a tautomer or an isomer mixture thereof, or a pharmaceutically acceptable salt thereof, or a prodrug thereof, or a metabolite thereof: 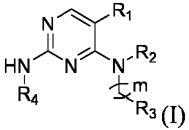
wherein R₁ is selected from one of halogen, trifluoromethyl, C₁-C₆ alkyl, or C₁-C₆ alkoxy;
R₂ is selected from any one of H or C₁-C₅ alkyl;
R₃ is selected from any of the following substituents: 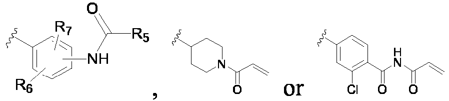
wherein R₆ is selected from Cl or methyl; R₇ is selected from H or Cl; R₅ is selected from vinyl, propenyl, butenyl, trifluorovinyl, methyl, ethyl, phenyl, tetrahydrofuran-2-yl, or acrylamide;
R₄ is selected from any of the following substituents: 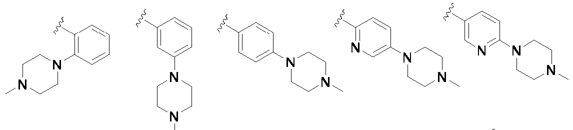 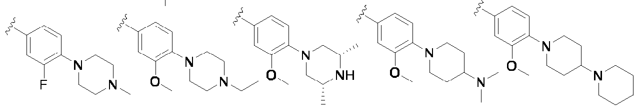 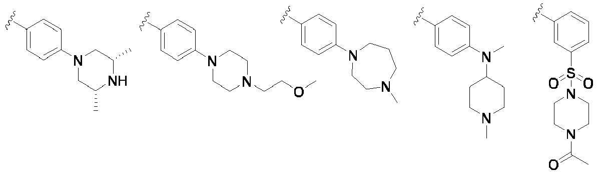
wherein m is 0 or 1 or 2, and 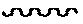 represents a substitution position.

2. The 2,4,5-trisubstituted pyrimidine compound according to claim 1, wherein the R₃ is selected from any of the following substituents:

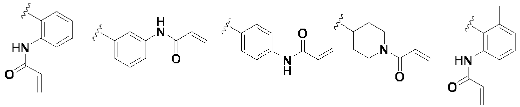

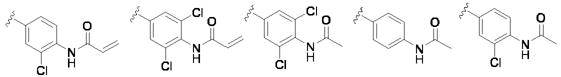

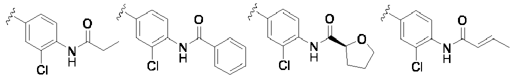

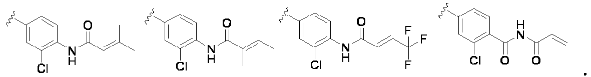

3. The 2,4,5-trisubstituted pyrimidine compound according to claim 1, wherein the R₁ is selected from F, Cl, CH₃, OCH₃ or CF₃.

4. The 2,4,5-trisubstituted pyrimidine compound according to claim 1, wherein the R₂ is H, CH₃ or C₂H₅.

5. The 2,4,5-trisubstituted pyrimidine compound according to claim 1, wherein the R₁ is Cl; and the R₂ is H;
m is 0;
the R₃ is 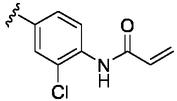
; and
the R₄ is selected from any of the following substituents:

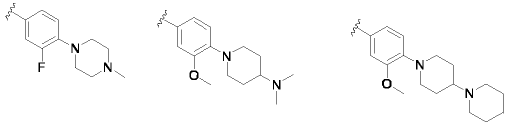

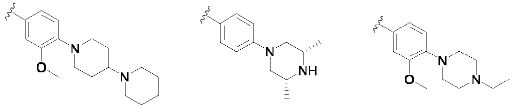

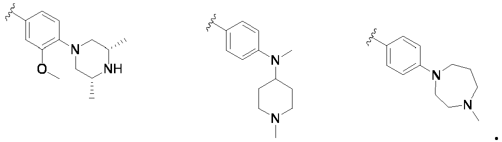

6. The 2,4,5-trisubstituted pyrimidine compound according to claim 1, wherein the compound is one of the following compounds, an isotope labeled compound thereof, or an optical isomer thereof, a geometric isomer, a tautomer or an isomer mixture thereof, or a pharmaceutically acceptable salt thereof, or a prodrug thereof, or a metabolite thereof:
N-(4-((5-fluoro-2-((4-(4-methylpiperazine-1-yl)phenyl)amino)pyrimidine-4-yl)amino)phenyl)acr ylamide (Compound 1);
N-(4-((5-chloro-2-((4-(4-methylpiperazine-1-yl)phenyl)amino)pyrimidine-4-yl)amino)phenyl)ac rylamide (Compound 2);
N-(4-((5-methyl-2-((4-(4-methylpiperazine-1-yl)phenyl)amino)pyrimidine-4-yl)amino)phenyl)ac rylamide (Compound 3);
N-(4-((5-methoxy-2-((4-(4-methylpiperazine-1-yl)phenyl)amino)pyrimidine-4-yl)amino)phenyl) acrylamide (Compound 4);
N-(2-((5-fluoro-2-((4-(4-methylpiperazine-1-yl)phenyl)amino)pyrimidine-4-yl)amino)phenyl)acr ylamide (Compound 5);
N-(2-((5-methyl-2-((4-(4-methylpiperazine-1-yl)phenyl)amino)pyrimidine-4-yl)amino)phenyl)ac rylamide (Compound 6);
N-(2-((5-methoxy-2-((4-(4-methylpiperazine-l-yl)phenyl)amino)pyrimidine-4-yl)amino)phenyl) acrylamide (Compound 7);
N-(2-((2-((4-(4-methylpiperazine-1-yl)phenyl)amino)-5-(trifluoromethyl)pyrimidine-4-yl)amino) phenyl)acrylamide (Compound 8);
N-(2-((5-chloro-2-((4-(4-methylpiperazine-1-yl)phenyl)amino)pyrimidine-4-yl)amino)phenyl)ac rylamide (Compound 9);
N-(2-((5-chloro-2-((4-(4-methylpiperazine-1-yl)phenyl)amino)pyrimidine-4-yl)amino)phenyl)ac rylamide (Compound 10);
N-(2-((5-chloro-2-((4-(4-methylpiperazine-1-yl)phenyl)amino)pyrimidine-4-yl)amino)-3-methyl phenyl)acrylamide (Compound 11);
N-(3-methyl-2-((5-methyl-2-((4-(4-methylpiperazine-1-yl)phenyl)amino)pyrimidine-4-yl)amino) phenyl)acrylamide (Compound 12);
N-(2-((5-methoxy-2-((4-(4-methylpiperazine-1-yl)phenyl)amino)pyrimidine-4-yl)amino)-3-meth ylphenyl)acrylamide (Compound 13);
N-(3-methyl-2-((2-((4-(4-methylpiperazine-1-yl)phenyl)amino)-5-(trifluoromethyl)pyrimidine-4-yl)amino)phenyl)acrylamide (Compound 14);
N-(3-((5-fluoro-2-((4-(4-methylpiperazine-1-yl)phenyl)amino)pyrimidine-4-yl)amino)phenyl)acr ylamide (Compound 15);
N-(3-((5-methyl-2-((4-(4-methylpiperazine-1-yl)phenyl)amino)pyrimidine-4-yl)amino)phenyl)ac rylamide (Compound 16);
N-(4-(2-((5-fluoro-2-((4-(4-methylpiperazine-1-yl)phenyl)amino)pyrimidine-4-yl)amino)ethyl)p henyl)acrylamide (Compound 17);
N-(4-(((5-fluoro-2-((4-(4-methylpiperazine-1-yl)phenyl)amino)pyrimidine-4-yl)amino)methyl)p henyl)acrylamide (Compound 18);
1-(4-((5-fluoro-2-((4-(4-methylpiperazine-1-yl)phenyl)amino)pyrimidine-4-yl)amino)piperidine-1-yl)prop-2-en-1-one (Compound 19);
N-((5-fluoro-2-((4-(4-methylpiperazine-1-yl)phenyl)amino)pyrimidine-4-yl)amino)phenyl)aceta mide (Compound 20);
N-(2,6-dichloro-4-((5-fluoro-2-((4-(4-methylpiperazine-1-yl)phenyl)amino)pyrimidine-4-yl)ami no)phenyl)acetamide (Compound 21);
N-(2,6-dichloro-4-((5-chloro-2-((4-(4-methylpiperazine-1-yl)phenyl)amino)pyrimidine-4-yl)ami no)phenyl)acetamide (Compound 22);
N-(2-chloro-4-((5-fluoro-2-((4-(4-methylpiperazine-1-yl)phenyl)amino)pyrimidine-4-yl)amino)p henyl)acetamide (Compound 23);
N-(2-chloro-4-((5-fluoro-2-((4-(4-methylpiperazine-1-yl)phenyl)amino)pyrimidine-4-yl)amino)p henyl)propionamide (Compound 24);
N-(2-chloro-4-((5-fluoro-2-((4-(4-methylpiperazine-1-yl)phenyl)amino)pyrimidine-4-yl)amino)p henyl)benzamide (Compound 25);
N-(2-chloro-4-((5-chloro-2-((4-(4-methylpiperazine-1-yl)phenyl)amino)pyrimidine-4-yl)amino)p henyl)acetamide (Compound 26);
N-(2-chloro-4-((5-fluoro-2-((4-(4-methylpiperazine-1-yl)phenyl)amino)pyrimidine-4-yl)(methyl) amino)phenyl)acetamide (Compound 27);
N-(2-chloro-4-(ethyl(5-fluoro-2-((4-(4-methylpiperazine-1-yl)phenyl)amino)pyrimidine-4-yl)am ino)phenyl)acetamide (Compound 28);
N-(2-chloro-4-(ethyl(5-fluoro-2-((4-(4-methylpiperazine-1-yl)phenyl)amino)pyrimidine-4-yl)am ino)phenyl)acetamide (Compound 29);
N-(2-chloro-4-((5-chloro-2-((4-(4-methylpiperazine-1-yl)phenyl)amino)pyrimidine-4-yl)amino)p henyl)acrylamide (Compound 30);
N-(2,6-dichloro-4-((5-chloro-2-((4-(4-methylpiperazine-1-yl)phenyl)amino)pyrimidine-4-yl)ami no)phenyl)acrylamide (Compound 31);
N-(3-((5-chloro-2-((4-(4-methylpiperazine-1-yl)phenyl)amino)pyrimidine-4-yl)amino)phenyl)ac rylamide (Compound 32);
N-(4-(2-((5-chloro-2-((4-(4-methylpiperazine-1-yl)phenyl)amino)pyrimidine-4-yl)amino)ethyl)p henyl)acrylamide (Compound 33);
(E)-N-(2-chloro-4-((5-chloro-2-((4-(4-methylpiperazine-1-yl)phenyl)amino)pyrimidine-4-yl)ami no)phenyl)but- 2-enamide (Compound 34);
N-(2-chloro-4-((5-chloro-2-((4-(4-methylpiperazine-1-yl)phenyl)amino)pyrimidine-4-yl)amino)p henyl)-3-methylbut-2 -enamide (Compound 35);
(E)-N-(2-chloro-4-((5-chloro-2-((4-(4-methylpiperazine-1-yl)phenyl)amino)pyrimidine-4-yl)ami no)phenyl)-2 -methylbut-2-enamide (Compound 36);
(E)-N-(2-chloro-4-((5-chloro-2-((4-(4-methylpiperazine-1-yl)phenyl)amino)pyrimidine-4-yl)ami no)phenyl)-4 ,4,4-trifluorobut-2-enamide (Compound 37);
N-acryloyl-2-chloro-4-((5-chloro-2-((4-(4-methylpiperazine-1-yl)phenyl)amino)pyrimidine-4-yl) amino)benzamide (Compound 38);
N-(2-chloro-4-((5-chloro-2-((3-(4-methylpiperazine-1-yl)phenyl)amino)pyrimidine-4-yl)amino)p henyl)acrylamide (Compound 39);
N-(2-chloro-4-((5-chloro-2-((2-(4-methylpiperazine-1-yl)phenyl)amino)pyrimidine-4-yl)amino)p henyl)acrylamide (Compound 40);
N-(2-chloro-4-((5-chloro-2-((6-(4-methylpiperazine-1-yl)pyridine-3-yl)amino)pyrimidine-4-yl)a mino)phenyl)acrylamide (Compound 41);
N-(2-chloro-4-((5-chloro-2-((4-((3R,5S)-3,5-dimethylpiperazine-1-yl)phenyl)amino)pyrimidine-4-yl)amino)phenyl) acrylamide (Compound 42);
N-(2-chloro-4-((5-chloro-2-((4-(4-ethylpiperazine-1-yl)-3-methoxyphenyl)amino)pyrimidine-4-y l)amino)phenyl)acrylamide (Compound 43); N-(2-chloro-4-((5-chloro-2-((4-(4-(2-methoxyethyl) piperazine -1-yl)phenyl)amino)pyrimidine-4-yl)amino)phenyl)acrylamide (Compound 44);
N-(2-chloro-4-((5-chloro-2-((5-(4-methylpiperazine-1-yl)pyridine-2-yl)amino)pyrimidine-4-yl)a mino)phenyl)acrylamide (Compound 45);
N-(2-chloro-4-((5-chloro-2-((4-((3R,5S)-3,5-dimethylpiperazine-1-yl)-3-methoxyphenyl)amino) pyrimidine-4-yl) )amino)phenyl)acrylamide (Compound 46);
N-(2-chloro-4-((5-chloro-2-((4-((3R,5 S)-3,5-dimethylpiperazine-1-yl)-3-methoxyphenyl)amino) pyrimidine-4-yl) )amino)phenyl)acrylamide (Compound 47);
N-(2-chloro-4-((5-chloro-2-((4-(4-methyl-1,4-diazepine- 1 -yl)phenyl)amino)pyrimidine-4-yl)ami no)phenyl)acrylamide (Compound 48);
N-(2-chloro-4-((5-chloro-2-((3-fluoro-4-(4-methylpiperazine-1-yl)phenyl)amino)pyrimidine-4-yl )amino)phenyl)acrylamide (Compound 49);
N-(4-((2-((3-((4-acetylpiperazine-1-yl)sulfonyl)phenyl)amino)-5-chloropyrimidine-4-yl)amino)-2-chlorophenyl)acrylamide (Compound 50);
N-(2-chloro-4-((5-chloro-2-((4-(4-(dimethylamino)piperidine-1-yl)-3-methoxyphenyl)amino)pyr imidine-4-yl)amino)phenyl)acrylamide (Compound 51); and
N-(4-((2-((4-([1,4'-bipiperidine]-1'-yl)-3-methoxyphenyl)amino)-5-chloropyrimidine-4-yl)amino )-2-chlorophenyl)acrylamide (Compound 52).

7. A pharmaceutical composition containing the 2,4,5-trisubstituted pyrimidine compound according to any one of claims 1-6, wherein the pharmaceutical composition is composed of the 2,4,5-trisubstituted pyrimidine compound and a pharmaceutical excipient; and
a dosage form of the pharmaceutical composition is any of an injection, tablet, capsule, aerosol, suppository, film, pellet, ointment, controlled-release formulation, sustained-release formulation, or nano-formulation.

8. A use of the 2,4,5-trisubstituted pyrimidine compound according to any one of claims 1-6 in the preparation of a drug, wherein the drug is used to treat diseases or conditions related to FGFR; and
the drug uses the 2,4,5-trisubstituted pyrimidine compound as an active ingredient.

9. The use according to claim 8, wherein the diseases or conditions related to FGFR are selected from cancers, bone disorders or chondrocyte disorders, hypophosphatemia disorders, and fibrotic diseases.

10. The use according to claim 9, wherein cancers related to FGFR are selected from one or more of hepatocellular carcinoma, breast cancer, bladder cancer, colorectal cancer, melanoma, mesothelioma, lung cancer, prostate cancer, pancreatic cancer, testicular cancer, thyroid cancer, squamous cell carcinoma, glioblastoma, fibrosarcoma, uterine cancer, rhabdomyosarcoma, skin cancer, kidney cancer, lymphoma, cervical cancer, head and neck squamous cell carcinoma, malignant hydatidiform mole, choriocarcinoma, ovarian cancer, gastric cancer, and leukemia.
